Europäisches Patentamt

European Patent Office

Office européen des brevets

(19)

(11) Numéro de publication: **0 162 735**
**B1**

(12) **FASCICULE DE BREVET EUROPEEN**

(45) Date de publication du fascicule du brevet: **28.11.90**

(51) Int. Cl.⁵: **A 61 F 7/00**

(21) Numéro de dépôt: **85400574.1**

(22) Date de dépôt: **25.03.85**

(60) **Demande divisionnaire 89112085.9 déposée le 25/03/85.**

(54) **Appareil d'examen et de localisation de tumeurs par ultrasons muni d'un dispositif de traitement localisé par hyperthermie.**

(30) Priorité: **03.05.84 FR 8406877**

(43) Date de publication de la demande: **27.11.85 Bulletin 85/48**

(45) Mention de la délivrance du brevet: **28.11.90 Bulletin 90/48**

(84) Etats contractants désignés: **BE CH DE GB IT LI NL SE**

(56) Documents cités:
**EP-A-0 068 961**
**EP-A-0 081 639**
**DE-A-2.722 252**
**GB-A-2 126 901**
**US-A-4 204 435**

**ULTRASONICS, vol. 5, avril 1967, pages 105-112, Guilford, Surrey, GB; P.P. LEILE: "Production of deep focal lesions by focused ultrasound-current status"**

(73) Titulaire: **Dory, Jacques**
**91 rue des Molveaux**
**F-77450 Coupvray Esblay (FR)**

(72) Inventeur: **Dory, Jacques**
**91 rue des Molveaux**
**F-77450 Coupvray Esblay (FR)**

(74) Mandataire: **Marquer, Francis et al**
**CABINET MOUTARD 35, avenue Victor Hugo**
**Résidence Champfleury**
**F-78180 Voisins-le-Bretonneux (FR)**

(56) Documents cités:
**MESURES.REGULATION.AUTOMATISME, vol. 45, février 1980, pages 25-29, Paris, FA; "Echographie ultrasonore: un circuit CCD pour simplifier l'electronique de commande"**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

## Description

Les appareils d'échographie classiques permettant évidemment de procéder à l'examen de tumeurs à l'intérieur du corps en en formant une image sur l'écran d'un tube cathodique.

On sait par ailleurs qu'il est possible d'obtenir une destruction des cellules—en particulier des cellules malignes—en les soumettent de façon plus ou moins prolongée à une élévation de température. Les cellules à détruire doivent par exemple être portées à environ 45°C et ce, de manière bien contrôlée, en évitant d'atteindre des températures excessives qui pourraient provoquer des brûlures graves autour de la lésion. Le problème technique à résoudre consiste donc à la fois à maîtriser la quantité d'énergie et sa localisation.

Les différents procédés antérieurs (emploi d'hyperfréquences, de rayonnement infra-rouge, et autres) permettent de traiter des tumeurs superficielles, mais non d'atteindre des tissus plus profonds.

Selon EP—A—68 961 un dispositif d'hyperthermie combine un générateur d'un faisceau focalisé d'ultrasons comportant un émetteur principal d'ondes électriques à haute fréquence et un transducteur piézo-électrique principal, à un dispositif d'échographie comprenant un générateur auxiliaire d'impulsions électriques à haute fréquence associé à un transducteur piézo-électrique auxiliaire et à des moyens de formation d'images de la zone à traiter.

Ce brevet ne prévoit pas d'interrompre l'émission principale pour pouvoir effectuer l'échographie pendant le traitement lui-même.

Par ailleurs, la focalisation du faisceau principal y est obtenue par déphasage électronique, et aucun balayage échographique n'est prévu.

L'invention propose de réaliser un appareil qui conjugue les trois fonctions de localisation de la zone à traiter, de traitement par élévation de température bien maîtrisée dans une région restreinte bien délimitée à l'intérieur de cette zone et de contrôle simultané des résultats du traitement.

A cet effet, l'invention de caractérisé par les caractéristiques de la seconde partie de la revendication 1.

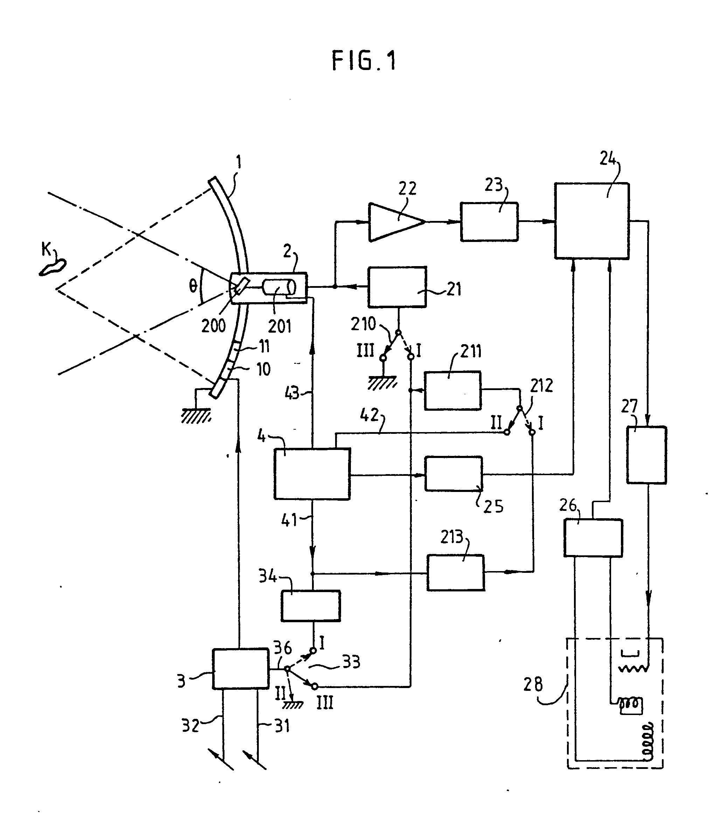

On notera que l'utilisation d'un transducteur à surface focalisante était connue en soi selon ULTRASONICS, vol. 5, avril 1967, figure 1c, tandis que le balayage échographique d'une zone à traiter était également connu en soi selon MESURES REGULATION AUTOMATISME, vol. 45, février 1980.

Il résulte de la revendication 4 que, pendant les modes auxiliaires de fonctionnement, destinés à effectuer des réglages précis, la qualité de l'image échographique, soit de la zone à traiter (mode de repérage), soit de la région focale (mode de contrôle de la région restreinte), sera sensiblement meilleure que pendant le mode de traitement, pendant lequel les images successives de la zone à traiter se succèderont par exemple à des intervalles de l'ordre de la seconde, ce qui permettra toutefois de vérifier de manière satisfaisante la position de la région focale pendant le traitement.

Suivant une forme d'exécution préférée, le transducteur principal est constitué par une mosaïque d'éléments piézo-électriques isolés les uns des autres et formant une calotte sphérique associée à des moyens de contrôler son déplacement suivant trois axes orthogonaux, tandis que le transducteur auxiliaire est solidarisé au sommet de ladite calotte et associé à des moyens d'effectuer un balayage sectoriel dans un plan qui passe par l'axe de symétrie de ladite calotte.

D'autres particularités, ainsi que les avantages de l'invention, apparaîtront clairement à la lumière de la description ci-après.

Au dessin annexé:

La figure 1 est le schéma de principe d'un appareil d'hyperthermie conforme à un mode d'exécution préféré de l'invention;

La figure 2 représente schématiquement, en perspective, le transducteur principal et son dispositif de support mobile;

La figure 3 représente les formes d'ondes en différents points des circuits de l'appareil; et

La figure 4 illustre l'image obtenue sur l'écran de visualisation que comporte l'appareil.

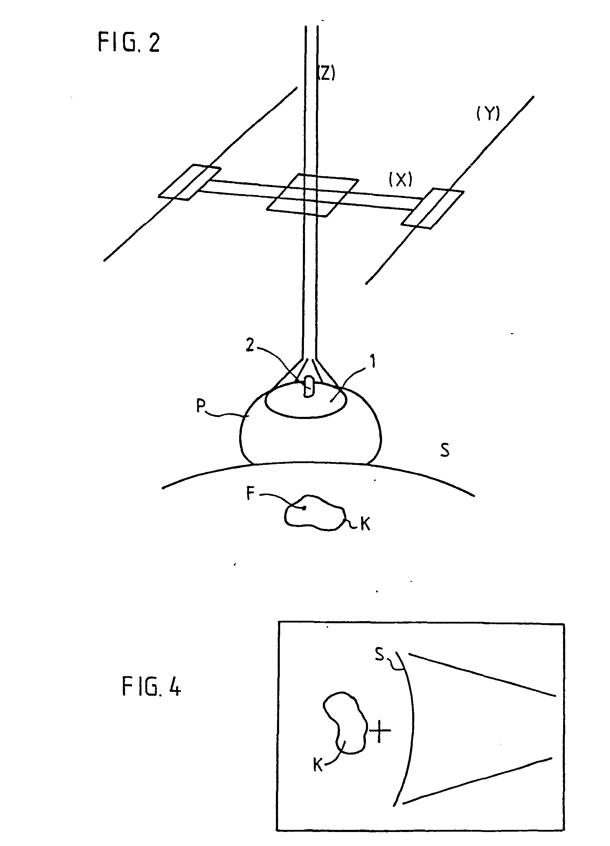

A la figure 2, on a représenté un transducteur principal 1 en forme de calotte sphérique supporté par un montage qui en permet les déplacements suivant trois axes orthogonaux X, Y et Z. Cet montage a été représenté de manière schématique, sa réalisation étant à la portée de l'homme du métier. Suivant l'axe de la calotte sphérique est disposé un transducteur auxiliaire 2 de forme générale cylindrique, qui passe à travers la calotte 1 et lui est fixé. Une poche d'eau P est interposée entre la calotte 1 et la surface S du corps du patient, celui-ci étant supposé allongé sur un plan horizontal.

La calotte 1 a par exemple 200 à 300 mm de diamètre et est composée d'un grand nombre (300 ou 400) d'éléments piézo-électriques 10, 11, etc... (figure 1) isolés les uns des autres et juxtaposés pour constituer une mosaïque. Ces éléments sont métallisés sur leurs deux faces, l'une des métallisations étant reliée à la masse et l'autre à des connexions d'excitation par un émetteur principal 3.

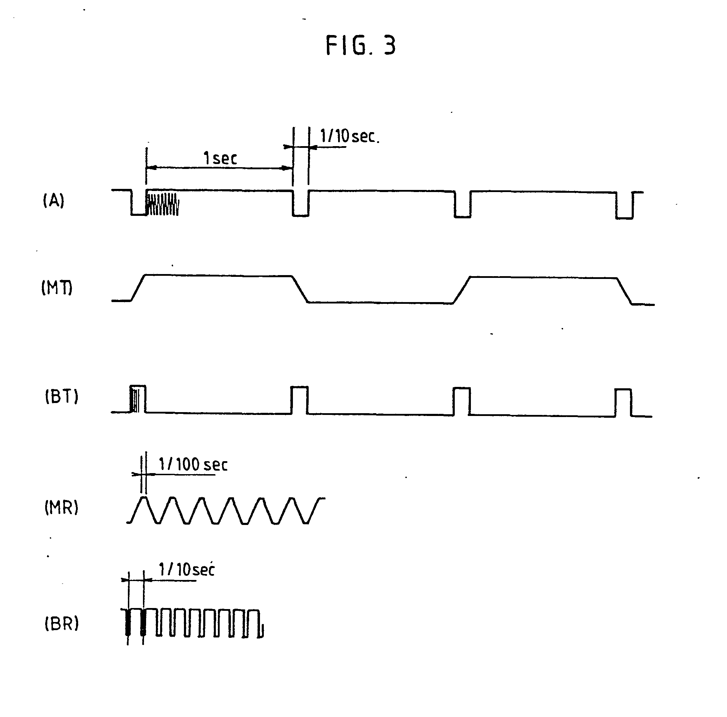

Ce dernier fournit un signal électrique A (figure 3) composé de trains d'ondes à haute fréquence (500 KHz par exemple) de puissance de crête relativement faible (une dizaine ou une centaine de watts par exemple), mais de durée relativement grande (par exemple de l'ordre de la seconde) séparés par des intervalles de temps de l'ordre de 1/10 sec., temps nécessaire pour la formation d'une image par le dispositif d'échographie. Il s'agit donc d'un régime d'émission quasi-continu, destiné au traitement. Un tel régime peut être obtenu au moyen d'émetteurs utilisant des transistors de puissance. De préférence, les éléments du transducteur 1 seront répartis en groupes excités chacun par un émetteur séparé (le rectangle 4 symbolisant l'ensemble de ces émetteurs), les éléments de chaque groupe étant répartis dans une même zone circulaire de la surface sphérique. En réglant les phases relatives des émissions, il est possible de modifier la répartition de l'énergie dans la région de focalisation du faisceau ultrasonore.

On a symbolisé par une entrée 31 de l'émetteur 3 un réglage de la puissance émise et par une entrée 32 un réglage de la durée des trains d'ondes. La tache focale formée au centre de la sphère pourra, avec cette technique, être très petite (diamètre de 2 ou 3 mm par exemple) et avoir une position rigoureusement fixe pour une position donnée du transducteur.

A la figure 1, on voit que le transducteur auxiliaire 2 est lui-même relié, d'une part, à un émetteur 21 d'impulsions électriques à haute fréquence, d'autre part, à un amplificateur de réception 22 suivi d'un convertisseur analogique-numérique 23, lui-même suivi d'une mémoire 24. L'émetteur 21 est synchronisé par un générateur d'impulsions 211 qui fournit 256 impulsions pendant chacun des intervalles de temps successifs de 1/10 de seconde. A chacun de ces intervalles de temps correspond un balayage complet d'un secteur angulaire prédéterminé θ (figure 1) par le faisceau émis par le transducteur 2, donc la formation, dans le plan de balayage, d'une image de la zone observée par le dispositif d'échographie.

Le transducteur 2 est avantageusement du type décrit dans les demandes de brevet français No 80 16717 déposée le 29 Juillet 1980 pour: "Sonde d'échographie à balayage sectoriel comportant deux liquides de couplage" et No 80 16718 déposée le 29 Juillet 1980 pour: "Sonde d'échographie à balayage sectoriel mécanique", c'est-à-dire qu'il comporte un élément piéco-électrique oscillant 200 commandé par un moteur 201, lui-même commandé par un circuit électronique que l'on a symbolisé par un rectangle 4. Ce circuit électronique fournit des signaux de commande du moteur 201 logé à l'intérieur du boîtier du transducteur 2 et est agencé de façon qu'une oscillation complète du moteur corresponde à la durée de formation d'une image définie ci-dessus (1/10 sec.).

Dans un premier mode de fonctionnement (traitement et contrôle) le commutateur 210 est en position I ainsi que les commutateurs 212 et 33.

Dans la position I des commutateurs 33 et 212, le générateur 211 est synchronisé par une première sortie 41 du circuit 4, et celui-ci est alors réglé, par des moyens non figurés, pour engendrer, sur sa sortie 43 reliée au moteur 201, des signaux ayant la forme d'onde (MT) représentée à la figure 4. Le balayage d'une image s'effectue donc en 1/10 s et est suivi d'un intervalle de temps de 1 s pendant lequel l'élément oscillant 200 reste immobile, si bien que le transducteur 2 ne reçoit pas d'échos.

Pendant les intervalles entre les périodes de balayage, un circuit 34 engendre des créneaux de 1 s qui servent à synchroniser l'émetteur 3, tandis que, pendant les périodes de balayage, un circuit 213 engendre des créneaux de 1/10 s qui servent à synchroniser le générateur 211.

Ainsi, dans ce mode de fonctionnement, le transducteur 1 engendre un faisceau d'ultrasons en régime quasi-continu, tandis que le dispositif d'échographie forme une image toutes les secondes dans les intervalles entre les trains d'ondes. On a représenté en (BT) la forme d'ondes des signaux alors émis par le générateur 211.

Dans un second mode de fonctionnement (repérage) le commutateur 210 restant en position I, le commutateur 33 est en position II, si bien que l'émetteur 3 n'est pas synchronisé et que le faisceau d'ultrasons focalisé n'est pas émis. Le commutateur 212 est également en position II, si bien que le générateur 211 est synchronisé par une deuxième sortie 42 du circuit 4 et celui-ci est réglé pour engendrer, sur sa sortie 43, des signaux ayant la forme d'onde (MR) représentée à la figure 3. Les balayages de 1/10 s sont donc séparés par des intervalles de temps de 1/100 sec. seulement et les images sont formées à partir des échos provenant de la réflexion des impulsions engendrées par le transducteur 2. Le générateur 211 fournit les signaux (BR).

Dans un troisième mode de fonctionnement (contrôle de la région focale), le commutateur 210 est en position III, si bien que l'émetteur 21 et le transducteur 2 n'émettent pas. Le commutateur 212 est encore en position II, si bien que le générateur 211 est synchronisé par la sortie 42 du circuit 4, et ce dernier est réglé comme dans le second mode de fonctionnement, si bien que les balayages de 1/10 s sont encore séparés par des intervalles de 1/100 sec. Le commutateur 33 est en position III et, par conséquent, l'émetteur 3 est maintenant synchronisé par le générateur 211 qui fournit alors les signaux (BR).

Dans ce troisième mode de fonctionnement, le dispositif échographique est donc constitué par l'émetteur 3, le transducteur 1 fonctionnant à l'émission et le transducteur 2 fonctionnant à la réception. Il en résulte qu'une image de la zone de concentration de l'énergie dans la région focale émise par le transducteur 1 est obtenue.

Les signaux échographiques reçus en 22 dans le premier ou le troisième modes de fonctionnement sont, après conversion analogique-numérique en 23, stockés ligne par ligne dans la mémoire 24, un dispositif d'adressage d'écriture 25, commandé par le circuit 4, permettant de faire correspondre les angles respectifs de déviation du faisceau émis et/ou reçu par le transducteur 2 aux lignes respectives de la mémoire. Un dispositif 26 de lecture rapide de la mémoire excite les bobines de déviation en X et en Y d'un tube cathodique 28, donc l'électrode de commande de brillance reçoit le contenu correspondant de la mémoire 24, transformé en signal analogique par un convertisseur numérique-analogique 27.

La réalisation pratique de tous les circuits décrits et représentés est à la portée de l'homme de l'Art. Le circuit de commande 4 pourra par exemple comporter un monovibrateur fournissant des créneaux de durée réglable à 1/100 s ou 1 s. suivant le mode de fonctionnement et des circuits de génération de tensions croissantes et décroissantes de durée 1/10 s., déclenchés par lesdits créneaux.

L'appareil qui vient d'être décrit fonctionne de la manière suivante:

Dans le mode de fonctionnement en repérage, l'opérateur recherche et localise la zone à traiter. Le dispositif de visualisation est agencé, de manière connue en soi, pour matérialiser sur l'écran du tube cathodique (par exemple par une croix) la position théorique de la tache focale dans le plan de coupe représenté, plan qui passe par l'axe de symétrie du transducteur 1. (Il s'agit d'échographie du type B). L'opérateur commence par déplacer le transducteur 1 en X, jusqu'à ce que la tumeur apparaisse nettement sur l'écran, puis il le déplace en Y et Z, jusqu'à ce que la croix coïncide avec la région centrale de l'image de la tumeur (K, figure 4). A ce moment, les commutateurs peuvent être mis en position de contrôle de la région focale: seule celle-ci est alors rendue visible sur l'écran, avec une luminosité proportionnelle à la concentration d'énergie correspondante. On a ainsi une représentation de ce que sera la répartition de l'énergie de l'onde de traitement, ce qui permet de contrôler et de parfaire les réglages.

Pendant le traitement, l'appareil ne fournit qu'une image par seconde, mais cette cadence est suffisante pour permettre une vérification quasi permanente de la position de la tache focale.

Il est clair que l'appareil décrit permet le contrôle de l'évolution de la tumeur après chaque séquence de traitement.

**Revendications**

1. Appareil de traitement par hyperthermie combinant un générateur d'un faisceau focalisé d'ultrasons comportant un émetteur principal (3) d'ondes électriques à haute fréquence et un transducteur piézo-électrique principal (1), à un dispositif d'échographie comprenant un générateur auxiliaire d'impulsions électriques à haute fréquence (21—211) associé à un transducteur piézo-électrique auxiliaire (2) générant un faisceau ultrasonore d'examen et à des moyens de formation d'images de la zone à traiter, caractérisé en ce que la surface active du transducteur principal (1) est focalisante, que des moyens (200—201—4) sont prévus pour provoquer un balayage de formation d'images de la zone à traiter par ledit faisceau ultrasonore d'examen, et que des moyens de commutation (210 à 213 et 33) et de réglage permettent de provoquer l'émission dudit faisceau focalisé par le transducteur principal excité par l'émetteur principal sous la forme de trains d'ondes périodiquement séparés par des blancs de durée plus courte que lesdits trains d'ondes pendant un mode principal de fonctionnement en traitement et contrôle, lesdits moyens de commutation et de réglage provoquant l'émission du faisceau d'examen et la formation d'images échographiques pendant lesdits blancs.

2. Appareil selon la revendication 1, caractérisé en ce que les intervalles de temps d'émission du faisceau focalisé en mode traitement et contrôle sont de l'ordre de la seconde et sont séparés par des intervalles de formation d'images de l'ordre du dixième de seconde.

3. Appareil selon la revendication 1, caractérisé en ce que le transducteur principal est constitué par une mosaïque d'éléments piézo-électriques isolés les uns des autres et formant une calotte sphérique (1) associée à des moyens de contrôler son déplacement suivant trois axes orthogonaux (X, Y, Z), tandis que le transducteur auxiliaire (2) est solidarisé au sommet de ladite calotte et associé à des moyens (200—201) d'effectuer un balayage sectoriel dans un plan qui passe par l'axe de symétrie de ladite calotte.

4. Appareil de traitement par hyperthermie combinant un générateur d'un faisceau focalisé d'ultrasons comportant un émetteur principal (3) d'ondes électriques à haute fréquence et un transducteur piézo-électrique principal (1), à un dispositif d'échographie comprenant un générateur auxiliaire d'impulsions électriques à haute fréquence (21—211) associé à un transducteur piézo-électrique auxiliaire (2) générant un faisceau ultrasonore d'examen et à des moyens de formation d'images de la zone à traiter, caractérisé en ce que la surface active du transducteur principal (1) est focalisante, que des moyens (200—201—4) sont prévus pour provoquer un balayage de formation d'images de la zone à traiter par ledit faisceau ultrasonore d'examen, que des moyens de commutation (210 à 213 et 33) et de réglage permettent de provoquer l'émission dudit faisceau focalisé par le transducteur principal excité par l'émetteur principal sous la forme de trains d'ondes périodiquement séparés par des blancs de durée plus courte que lesdits trains d'ondes, pendant un mode principal de fonctionnement en traitement et contrôle, au cours duquel lesdits moyens de commutation et de réglage provoquent l'émission du faisceau d'examen et la formation d'images échographiques pendant lesdits blancs, que pendant un second mode de fonctionnement auxiliaire en repérage, lesdits moyens de commutation et de réglage provoquent seulement l'émission périodique du faisceau d'examen par le transducteur auxiliaire et la formation d'images, et que pendant un troisième mode de fonctionnement auxiliaire en contrôle de la région focale, lesdits moyens de commutation et de réglage provoquent l'émission périodique du faisceau focalisé, l'émetteur principal (3) étant synchronisé par un circuit de synchronisation (211) du générateur auxiliaire pour fonctionner en émetteur d'échographie, tandis que les échos sont reçus par le transducteur auxiliaire, les intervalles de temps qui séparent les périodes d'émission successives pendant les deux modes auxiliaires de fonctionnement étant sensiblement plus faibles que les blancs qui séparent les périodes d'émission du faisceau focalisé pendant le mode principal.

5. Appareil selon la revendication 4, caractérisé en ce que, pendant les deux modes de fonctionnement auxiliaire, les intervalles de formation d'images de l'ordre du dixième de seconde sont séparés par des intervalles d'arrêt du balayage de l'ordre du 1/100 de seconde.

## Patentansprüche

1. Gerät zur hyperthermischen Behandlung, welches kombiniert: den Generator eines fokussierten Ultraschallwellenstrahls, mit einem Hauptsender (3) für elektrische Hochfrequenzwellen und einem piezoelektrischen Haupttransduktor (1); eine Echographievorrichtung, mit einem Hilfsgenerator für elektrische Hochfrequenzimpulse (21—211), der einem piezoelektrischen Hilfstransduktor (2) zugeordnet ist, welcher einen Untersuchungs-Ultraschallwellenstrahl abgibt; und Mittel zur Erzeugung von Bildern der zu behandelnden Zone, dadurch gekennzeichnet, dass die aktive Fläche des Haupttransduktors (1) fokussiert, dass Mittel (200—201—4) vorgesehen sind, damit besagter Untersuchungs-Ultraschallwellenstrahl die zu behandelnde Zone zwecks Formung von Bildern abtastet und dass Umschalt—(210 bis 213 und 33) und Einstellmittel es ermöglichen, die Abgabe des besagten fokussierten Strahls durch den vom Hauptsender erregten Haupttransduktor zu bewirken, in Form von Wellenzügen, die in bestimmten Abständen durch Leerintervalle kürzerer Dauer als die Wellenzüge während einer Hauptbetriebsweise zur Behandlung und Kontrolle getrennt sind, und besagte Umschalt- und Einstellmittel die Abgabe des Untersuchungsstrahls und die Formung von Echographiebildern während besagter Leerintervalle bewirken.

2. Gerät nach Anspruch 1, dadurch gekennzeichnet, dass die Zeitintervalle der Abgabe des fokussierten Strahls während der Behandlungs- und Kontrollbetriebsweise in der Grössenordnung von einer Sekunde liegen und durch Intervalle zur Formung von Bildern in der Grössenordnung von 1/10 Sekunde getrennt sind.

3. Gerät nach Anspruch 1, dadurch gekennzeichnet, dass der Haupttransduktor aus einem Mosaik von im Verhältnis zueinander isolierten piezoelektrischen Elementen besteht, die eine kugelförmige Schale (1) bilden, welche Mitteln zugeordnet ist, um ihre Verschiebung gemäss drei senkrecht zueinander angeordneten Achsen (X, Y, Z) zu kontrollieren, während der Hilfswandler (2) fest mit dem Scheitelpunkt der Schale verbunden und Mitteln (200—201) zugeordnet ist, die eine abschnittsweise Abtastung in einer Ebene vornehmen, welche sich durch die Symmetrieachse der besagten Schale erstreckt.

4. Gerät zur hyperthermischen Behandlung, welches kombiniert: den Generator eines fokussierten Untraschallwellenstrahls, mit einem Hauptsender (3) für elektrische Hochfrequenzwellen und einem piezoelektrischen Haupttransduktor (1); eine Echographievorrichtung, mit einem Hilfsgenerator für elektrische Hochfrequenzimpulse (21—211), der einem piezoelektrischen Hilfstransduktor (2) zugeordnet ist, welchen einen Untersuchungs-Ultraschallwellenstrahl abgibt;

und Mittel zur Erzeugung von Bildern der zu behandelnden Zone, dadurch gekennzeichnet, dass die aktive Fläche des Haupttransduktors (1) fokussiert, dass Mittel (200—20—4) vorgesehen sind, damit besagter Untersuchungs-Ultraschallwellenstrahl die zu behandelnde Zone zwecks Formung von Bildern abtastet, dass Umschalt- (210 bis 213 und 33) und Einstellmittel es ermöglichen, die Abgabe des besagten fukussierten Strahls durch den vom Hauptsender erregten Haupttransduktor zu bewirken, in Form von Wellenzügen, die in bestimmten Abständen durch Leerintervalle kürzerer Dauer als die Wellenzüge während einer Hauptbetriebsweise zur Behandlung und Kontrolle getrennt sind, während derer besagte Umschalt- und Einstellmittel die Abgabe des Untersuchungsstrahls und die Formung von Echographiebildern während besagter Leerintervalle bewirken, dasss, während einer zweiten Hilfsbetriebsweise zur Ortung, besagte Umschalt- und Einstellmittel nur die periodische Abgabe des Untersuchungsstrahls durch den Hilfswandler und die Formung von Bildern bewirken, und dass, während einer dritten Hilfsbetriebsweise zur Kontrolle des Fokalbereichs, besagte Umschalt- und Einstellmittel die periodische Abgabe des fokussierten Strahls bewirken, wobei der Hauptsender (3) durch einen Synchronisierkreis (211) des Hilfsgenerators synchronisiert ist, um als Echographiesender zu arbeiten, während die Echos vom Hilfstransduktor empfangen werden und die Zeitintervalle, welche die aufeinanderfolgenden Abgabeperioden während der beiden Hilfsbetriebsweisen trennen, wesentliche geringer sind, als die Leerintervalle, welche die Abgabeperioden des fokussierten Strahls in der Hauptbetriebsweise trennen.

5. Gerät nach Anspruch 4, dadurch gekennzeichnet, dass, während der beiden Hilfsbetriebsweisen, die Intervalle in der Grössenordnung von 1/10 Sekunde in denen Bilder geformt werden, durch Intervalle in der Grössenordnung von 1/100 Sekunde, in denen die Abtastung aussetzt, getrennt sind.

## Claims

1. A hyperthermia treatment apparatus, combining a generator of a focused ultrasonic beam comprising a main high frequency electric wave emitter (3) and a main piezoelectric transducer (1) with an echography device comprising an auxiliary high frequency electric pulse generator (21—211) associated with an auxiliary piezoelectric transducer (2) generating an ultrasonic examination beam and with means for forming images of the zone to be treated, characterized in that the active surface of the main transducer (1) is focusing, that means (200—201—4) are provided for causing the zone to be treated to be swept over by the said ultasonic examination beam in order to form images, and in that switching (210 to 213 and 33) and adjustment means cause emission of the said focused beam by the main transducer, energized by the main emitter in the form of wave trains periodically separated by blanks of a duration shorter than the said wave trains, during a main treatment and checking operating mode, said switching and adjustment means causing emission of the examination beam and formation of echographic images during said blanks.

2. The apparatus as claimed in claim 1, characterized in that the time intervals for emitting the focused beam in the treatment and checking mode are of the order of a second and are separated by image formation intervals of the order of a tenth of a second.

3. The apparatus as claimed in claim 1, characterized in that said main transducer is formed by a mosaic of piezoelectric elements isolated from each other and forming a spherical skull cap (1), associated with means for controlling its movement along three orthogonal axes (X, Y, Z), whereas the auxiliary transducer (2) is fixed to the top of said skull cap and is associated with means (200—201) for providing a sectorial sweep in a plane which passes through the axis of symmetry of said skull cap.

4. A hyperthermia treatment apparatus, combining a generator of a focused ultrasonic beam comprising a main high frequency electric wave emitter (3) and a main piezoelectric transducer (1) with an echography device comprising an auxiliary high frequency electric pulse generator (21—211) associated with an auxiliary piezoelectric transducer (2) generating an ultrasonic examination beam, and with means for forming images of the zone to be treated, characterized in that the active surface of the main transducer (1) is focusing, in that means (200—201—4) are provided for causing the zone to be treated to be swept over by the said ultrasonic examination beam, in order to form images, in that switching (210 to 213 and 33) and adjustment means cause emission of the said focused beam by the main transducer energized by the main emitter in the form of wave trains periodically separated by blanks of a duration shorter than the said wave trains, during a main treatment and checking operating mode during which said switching and adjustment means cause emission of the examination beam and formation of echographic images during said blanks, in that during a second auxiliary locating operating mode, asid switching and adjustment means only cause periodic emission of the examination beam by the auxiliary transducer and the formation of images, and in that, during a third auxiliary operating mode for checking the focal region, said switching and adjustment means cause periodic emission of the focused beam, the main emitter (3) being synchronized by a synchronization circuit (211) of the auxiliary generator for echographic operation, whereas the echoes are received by the auxilairy transducer, the time intervals which separate the successive emission periods during the two auxiliary operating modes being substantially smaller than the blanks which separate the periods of emission of the focused beam during the main mode.

5. The apparatus as claimed in claim 4, characterized in that, during the two auxiliary operating modes, the image-formation intervals of the order of a tenth of a second are separated by intervals of the order of 1/100 second during which the sweep is stopped.

# FIG.1

FIG. 2

(Z)

(Y)

(X)

2

1

P

S

F

K

FIG. 4

S

K

+

# FIG. 3